# EUROPEAN PATENT SPECIFICATION

(11) **EP 1 694 709 B1**
(45) Date of publication and mention of the grant of the patent: **15.12.2010**
(21) Application number: 04819218.1
(22) Date of filing: 25.11.2004
(51) Int. Cl.: C07K 19/00, A61P 35/00, C07K 14/195

(54) **RECOMBINANT ANTI-CD64-ETA' IMMUNOTOXINS**
REKOMBINANTE ANTI-CD64-ETA' IMMUNOTOXINE
IMMUNOTOXINES ANTI-CD64-ETA' RECOMBINANTES

(30) Priority: 26.11.2003 EP 03027161
(43) Date of publication of application: 30.08.2006
(73) Proprietor: Fraunhofer-Gesellschaft zur Förderung der angewandten Forschung e.V., 80686 München (DE)
(72) Inventor: HUHN, Michael, 51375 Leverkusen (DE); FISCHER, Rainer, 52156 Monschau (DE); TUR, Mehmet Kemal, 50765 Köln (DE); THEPEN, Theo, NL-3741 BG Baarn (NL); BARTH, Stefan, 52159 Roetgen (DE)
(74) Representative: von Kreisler Selting Werner
(86) International application number: PCT/EP2004/013374
(87) International publication number: WO 2005/052007

(56) References cited:
- WO-A-00/28050
- WO-A-02/058728
- WO-A2-01/80880
- ZHONG R K ET AL: "Cytotoxicity of anti-CD64-ricin a chain immunotoxin against human acute myeloid leukemia cells in vitro and in SCID mice." JOURNAL OF HEMATOTHERAPY & STEM CELL RESEARCH. UNITED STATES FEB 2001, vol. 10, no. 1, February 2001 (2001-02), pages 95-105, XP009032950 ISSN: 1525-8165 cited in the application
- BARTH STEFAN ET AL: "Recombinant anti-CD25 immunotoxin RFT5(scFv)-ETA' demonstrates successful elimination of disseminated human Hodgkin lymphoma in SCID mice" INTERNATIONAL JOURNAL OF CANCER, vol. 86, no. 5, 1 June 2000 (2000-06-01), pages 718-724, XP002286908 ISSN: 0020-7136
- BARTH S ET AL: "Compatible-solute-supported periplasmic expression of functional recombinant proteins under stress conditions" APPLIED AND ENVIRONMENTAL MICROBIOLOGY, vol. 66, no. 4, April 2000 (2000-04), pages 1572-1579, XP002286907 ISSN: 0099-2240
- HUHN ET AL: "Highly efficient periplasmic expression of functional Pseudomonas Exotoxin-based immunotoxins" ANNALS OF HEMATOLOGY, BERLIN, DE, vol. 77, no. SUPPL 2, 25 October 1998 (1998-10-25), pages S84-Abstr331, XP002099619 ISSN: 0939-5555
- SUNDARAPANDIYAN KARUNA ET AL: "Bispecific antibody-mediated destruction of Hodgkin's lymphoma cells" JOURNAL OF IMMUNOLOGICAL METHODS, vol. 248, no. 1-2, 1 February 2001 (2001-02-01), pages 113-123, XP002286909 ISSN: 0022-1759
- MATTHEY B ET AL: "A new series of pET-derived vectors for high efficiency expression of Pseudomonas exotoxin-based fusion proteins" GENE, ELSEVIER BIOMEDICAL PRESS. AMSTERDAM, NL, vol. 229, no. 1-2, 18 March 1999 (1999-03-18), pages 145-153, XP004161169 ISSN: 0378-1119
- BERA TAPAN K ET AL: "Bivalent disulfide-stabilized fragment variable immunotoxin directed against mesotheliomas and ovarian cancer" MOLECULAR CANCER THERAPEUTICS, vol. 1, no. 2, December 2001 (2001-12), pages 79-84, XP002287029 ISSN: 1535-7163 cited in the application
- SIEGALL C B ET AL: "CYTOTOXICITY OF CHIMERIC HUMAN-MURINE MONOCLONAL ANTIBODY BR96 IGG FAB'-2 AND FAB' CONJUGATED TO PSEUDOMONAS EXOTOXIN" BIOCONJUGATE CHEMISTRY, vol. 3, no. 4, 1992, pages 302-307, XP000606028 ISSN: 1043-1802
- CARTER PAUL: "Improving the efficacy of antibody-based cancer therapies." NATURE REVIEWS CANCER, vol. 1, no. 2, November 2001 (2001-11), pages 118-129, XP009033015 ISSN: 1474-175X (ISSN print) cited in the application
- GRAZIANO R.F. ET AL: 'Construction and characterization of a humanized anti-gamma-Ig receptor type I (Fc gamma RI) monoclonal antibody' J IMMUNOL. vol. 155, no. 10, 15 November 1995, page 4996-5002

## Description

### Technical Field

The present invention relates generally to the treatment of diseases involving CD64, and more specifically, to the preparation and use of recombinant complexes containing CD64-specific ligands, which induce internalization of the complex after binding, and also cytotoxic agents to alter the function, gene expression, or viability of a cell in a therapeutic manner.

### Background of the invention

Medications currently available for the treatment of a wide variety of diseases, such as chemotherapeutic agents, corticosteroids and unspecific immunosuppressive agents, have the disadvantage of potentially inducing some general damage, due to their relative non-specificity. It has been attempted to moderate this by various therapeutic concepts. One alternative approach is the use of immunotherapeutic agents to increase the specificity of medicaments. This approach has especially been described for the treatment of tumours.

If the immunotherapeutic agent is an immunotoxin then a monoclonal antibody (moAb) or an antibody fragment, which has a specific affinity for e.g. surface markers of tumour cells, is coupled with a cytotoxic reagent. As cytotoxic reagents toxins and radioactivity have been used. A direct therapeutic effect on the target cells was achieved with antibodies linked to radioactive elements or toxins to form so-called radioimmunoconjugates or immunotoxins (ITs). When radioactively labelled anti-B-cell moAb were used with B-cell lymphomas, tumour regressions and even complete remissions were observed (1). In contrast, the results with moAb against solid tumours were rather disillusioning. The relative large size of the ITs used in these studies seemed to interfere with their ability to penetrate the tumours, and made them ineffective therapeutics. The low tumour penetration rate posed a particular challenging problem for poorly vascularized tumours. In order to obtain better tissue and tumour penetration and in general improved diffusion properties, the ITs were miniaturized. It was also speculated, that these smaller ITs would be less immunogenic because of the reduced size of the antigenic determinants (2). Therefore proteolytically cleaved antibody fragments (miniaturized) were conjugated to the above mentioned effector functions (radioactive elements or toxins).

Improved cloning techniques allowed the preparation of completely recombinant ITs: coding regions of immunoglobulin light and heavy chain variable regions, amplified by polymerase chain reaction, were joined together by a synthetic linker e.g. (Gly₄Ser)₃. The resulting single chain fragment of variable region genes (scFv) was then genetically fused to a coding region of a catalytically active enzyme including cytotoxically active proteins or polypeptides (3).

The peptidic cell poisons which have been mostly used to date and thus best characterized are the bacterial toxins diphtheria toxin (DT), *Pseudomonas* exotoxin A (PE), and the plant-derived Ricin-A (4). The mechanism of cytotoxic activity is essentially the same in all of these toxins despite of their different evolutionary backgrounds. The catalytic domain inhibits protein biosynthesis by direct modification of the elongation factor 2 (EF-2), which is important to translation, or by inactivation of the EF-2 binding site at the 28S-rRNA subunit of ribosomes.

In most of the constructs employed to date, the systemic application of immunotoxins results in more or less severe side effects. In addition to the "vascular leak" syndrome, thrombocytopenia, hemolysis, renal insufficiency and sickness also occur, depending on the construct employed and the applied dosage (4). Dose-dependent liver damage was also observed (5). In addition to the documented side effects, the immunogenicity of the constructs is one of the key problems of immunotherapy. This applies, in particular, to the humoral defence against the catalytic domains employed, such as ricin (HARA), PE, or DT (2). Theoretically, all non-human structures can provoke an immune response. Thus, the repeated administration of immunotoxins and immunoconjugates is limited. A logical consequence of these problems is the development of human immunotoxins.

To date, human toxins used in immunotoxins have in most of all cases been selected from ribonucleases (6). Since human RNases are present in extracellular fluids, plasma and tissues, they are considered less immunogenic when used in immunotoxins. Angiogenin (ANG), a 14 kDa protein having a 64% sequence homology with RNase A, was first isolated from a tumour-cell-conditioned medium, where it was discovered due to its capability of inducing angiogenesis (7). It was shown that the t-RNA-specific RNase activity of Angiogenin has a cytotoxic potential. In accordance with that, chemically conjugated immunotoxins subsequently exhibited a cell-specific toxic activity. To evaluate the effectiveness of ANG-based immunotoxins, different conformations of ANG with e.g. epidermal growth factor (EGF) or CD30 ligand were constructed and successfully tested *in vitro* (8). Another member of the RNase superfamily is eosinophilic neurotoxin (EDN). For EDN, which has a size of 18.4 kDa, only the direct neurotoxicity has been described to date. Based on the documented potency, different EDN-based immunotoxins have been constructed and successfully tested *in vitro* (9).

Very recently it was shown that proteases like granzyme B or derivatives thereof can efficiently fulfil the effector function of immunotoxins (PCT/EP01/04514).

CD64 is a 72 kDa cell surface glycoprotein, which is normally expressed on monocytes/macrophages and dendritic cells (10). The biological functions mediated by this receptor include superoxide and cytokine production (TNF-α, IL-1, IL-6), cytotoxicity, endocytosis/phagocytosis and support of antigen presentation. This receptor represents an appropriate target for immunotherapy of hematologic malignancies because it is not present on stem cells thus guaranteeing regeneration of normal CD64-positive immune effector cells (11).

The ultimate goal in the treatment of cancer patients is the elimination of every tumour cell. Patients with acute myeloid leukemia (AML) have a total of 10¹² to 10¹³ malignant cells at the time of diagnosis. Per definition, complete remission is achieved after therapy as soon as less than 5% of malignant cells are detectable in the bone marrow. However, these patients still may carry as much as 10¹⁰ malignant cells in the blood stream at this moment. These, clinically unidentifiable minimal residual cells are the most common cause of relapse (12). Despite advances in polychemo- and radiotherapy, only about 20%-30% of patients with AML achieve long-term disease-free survival after first-line therapy. Thus, the elimination of minimal residual disease (MRD) might improve the outcome of patients with AML. Selective approaches, including antibody-based therapies, targeting cytotoxic agents to these cells might offer a promising tool for specific elimination of MRD. In order to improve the anti-tumour activity of native antibodies, drugs, isotopes and toxins have been conjugated to monoclonal antibodies (13).

Recently, a chemically-linked anti-CD64 immunotoxin showed rapid binding to and efficient internalization into CD64-positive leukemia cells *in vitro* and *in vivo* (14). The authors documented rapid tumour regression of tumour masses ranging from 85% to > 90% in a human AML model in NOD/SCID mice. The major obstacle observed in this and other trials were unspecific toxicities, mainly related to the vascular leak syndrome induced by Ricin-A-based chemically-linked toxins due to their unspecific binding to epithelial cells (13-15).

The problem associated with presently used, chemically-linked immunotoxins are their unspecific toxicity, size, and composition, resulting in a reduced efficiency of the therapeutic agent.

In contrast to Sera et al., 2001 (15) it is disclosed that by increasing the valency of the binding ligand, a significant increase in the *in vivo* cytotoxicity of the immunotoxin is achieved in a chronic inflammatory mouse model. The bivalent immunotoxins can also be used in the field of treating (malignant) diseases such as acute myeloid leukemia, arthritis, chronic obstructive pulmonary disease (COPD); including e.g.: emphysema, intrinsic and extrinsic asthma, cutaneous disease including e.g.: atopic dermatitis, polymorphic light eruption, systemic lupus erythematosus (SLE), or autoimmune diseases e.g.: graft versus host, multiple sclerosis, macrophage activation syndrome, rheumatoid arthritis, juvenile arthritis, intestinal diseases; including e.g.: Crohn's disease, chronic bowel disease.

R.K. Zhoug in JOURNAL OF HEMATOTHERAPY & STEM CELL RESEARCH 10:95-105 (2001) disclose that blast cells from patients with acute myeloid leukemia (AML) commonly express CD64, the high-affinity receptor for immunoglobulin G (FcγRI). An immunotoxin (MDX-44) was constructed by coupling humanized anti-CD64 monoclonal antibody (mAb) H22 via a bivalent linker to deglycosylated ricin A-chain (RA). Human leukemia cell lines were incubated with MDX-44 or H22/free RA. The effect of MDX-44 on the proliferation of leukemia cells was assessed by [³H]thymidine incorporation. In the presence of interferon-γ (IFN-γ), MDX-44 significantly inhibited the proliferation of CD64⁺ HL-60, NB4, and U937 cells in 72-h cultures in a dose-dependent manner. The mechanism of action appeared to be the induction of apoptosis, as measured by propidium iodide staining and flow cytometry analysis. However, CD64⁻ KG-1a and Daudi cells were not affected by MDX-44/IFN-γ. Incubating HL-60 cells with MDX-44/IFN-γ resulted in a 99% decrease in colony-forming units, whereas colony-forming cells in normal bone marrow were not significantly suppressed by such treatment. Cells from 60% of AML patients (6/10) were inhibited by MDX-44/IFN-γ, and the inhibition was correlated with CD64 expression on these cells (*r* = 0.65). In a human AML model in NOD/SCID mice, MDX-44/IFN-γ inhibited 95-98% of peritoneal exudate AML cell proliferation and 85-90% of solid leukemia masses. The effect of MDX-44 on AML cells was dependent on activation of cells by IFN-γ. MDX-44/IFN-γ may have value in the therapy of AML cells expressing cell-surface CD64.

S. Barth et al. in APPLIED AND ENVIRONMENTAL MICROBIOLOGY, Apr. 2000, p. 1572-1579 disclose a novel strategy for the expression of functional rIT directed to the periplasmic space of *Escherichia coli.* rITs were recovered by freeze-thawing of pellets from shaking cultures of bacteria grown under osmotic stress (4% NaCl plus 0.5 M sorbitol) in the presence of compatible solutes. Compatible solutes, such as glycine betaine and hydroxyectoine, are low-molecular-weight osmolytes that occur naturally in halophilic bacteria and are known to protect proteins at high salt concentrations. Adding 10 mM glycine betaine for the cultivation of *E*. *coli* under osmotic stress not only allowed the bacteria to grow under these otherwise inhibitory conditions but also produced a periplasmic microenvironment for the generation of high concentrations of correctly folded rITs. Protein purified by combinations of metal ion affinity and size exclusion chromatography was substantially stabilized in the presence of 1 M hydroxyecotine after several rounds of freeze-thawing, even at very low protein concentrations. The binding properties and cytotoxic potency of the rITs were confirmed by competitive experiments. This novel compatible-solute-guided expression and purification strategy might also be applicable for high-yield periplasmic production of recombinant proteins in different expression systems.

B. Matthey et al in Gene 229(1999) 145-153 disclose the development of a new bacterial expression system for high-level production of rITs. They constructed a series of pET-based vectors for peIB-directed periplasmic secretion or cytoplasmic production under the control of the T7*lac* promoter. Expression in *Escherichia coli* BL21(DE3)pLysS allowed a tightly regulated isopropyl β-*d*-thiogalactopyranoside (IPTG) induction of protein synthesis. An enterokinase-cleavable poly-histidine cluster was introduced into this setup for purification by affinity chromatography. A major modification resulted from the insertion of a specifically designed multiple cloning site. It contains only rare restriction enzyme recognition sites used for cloning of immunoglobulin variable region genes, as well as unique *Sfi*I and *Not*I restriction sites for directed insertion of single-chain variable fragments (scFv) available from established bacteriophage systems. For this purpose, they deleted two naturally occurring internal *Sfi*I consensus sites in a deletion mutant of *Pseudomonas aeruginosa* exotoxin A (ETA'). Each single structural element of the new vector (promoter, leader sequence, purification tag, scFv sequence, selectable marker, and toxin gene) was flanked by unique restriction sites allowing simple directional substitution. The fidelity of IPTG induction and high-level expression were demonstrated using an anti-CD30 scFv (Ki-4) fused to ETA'. These data confirm a bacterial vector system especially designed for efficient periplasmic expression of ETA'-based fusion toxins.

WO-A- 01/80880 discloses a complex formed at least by component A and at least component B wherein component A carries a bonding activity for cellular surface structures and component B carries a protease, such a granzyme B, as effector function.

### Summary of the invention

The present invention discloses a recombinant complex formed from at least one component A, at least one component B and at least one supplementary component C, whereby component A comprises a binding domain for the cellular surface receptor CD64, and component B a cell killing domain. The binding domain of component A is selected from the group of actively binding molecules, consisting of antibodies or their derivatives or fragments, synthetic peptides or low molecular weight molecules, ligands, receptor binding molecules, and their derivatives, mutants or combinations thereof, which bind to CD64. It is further disclosed that the component A has higher valency for CD64 by comprising two or more identical and/or different binding domains for CD64.

Additionally disclosed is that component B of the complex directly alters the function, gene expression or viability of a cell in a therapeutic manner, and more particular it inactivates molecules responsible for protein biosynthesis or activates components of cell- inherent apoptosis, inducing apoptosis in cells defined through the binding of component A. Component B of the cell killing domain has cytotoxic properties or is at least a member of ADP-ribosylating enzymes, such as the *Pseudomonas* Exotoxin A, Diphtheria-, Cholera- or the Pertussis-, Botulinumtoxin, or a member of the ribosome-inactivating proteins such as Dianthin, Saporin, Bryodin, Gelonin, Ricin, Abrin, Pokeweed Antiviral Protein (PAP) or Restrictocin, or is a member of the RNases (Phosphodiesterases) such as the Bovine seminal RNase, Bovine RNase A, Bovine pancreatic RNase, Angiogenin, Eosinophil-derived Neurotoxin (EDN), Eosinophilic Cationic Protein (ECP), Onconase, or Bullfrog Lectin, or is a member of the Prodrug-activating enzymes such as Calicheamicin, Glucose Oxidase, Carboxypeptidase, Alkaline Phosphatase, Cytosindeaminase, beta-Glucosidase, beta-Glucuronidase, beta-Lactamase, Nitroreductase, Thymidinkinase or Purin Nukleosid Phosphorylase, or is a member of the cathepsin protease family, or a member of the calpains, or a member of the granzymes, or any derivative of the above mentioned proteins, or a combination thereof. In a further embodiment supplementary component C of the complex of present invention regulates the expression of the gene (s) encoding the complex. The component C may enable the purification of the complex or its components, support the proteolytic removal of component A from component B, or the internalization of the component B into the target cell or the translocation of component B into a subcellular compartment, and/or enable the intracellular activation of component B.

The present invention concerns the monovalent complex m22(scFv)-ETA' (SEQ ID NO: 7) and the bivalent complex m22(scFv)₂-ETA' (SEQ ID NO: 8). Also the nucleic acid molecules, DNA and/or RNA coding for anyone of the said complexes or for the individual components for the preparation of such a complex are embodiments of present invention; in particular nucleic acids with the SEQ ID Nos: 5 and 6 encoding the monovalent complex m22(scFv)-ETA' (SEQ ID NO: 7) and the bivalent complex m22(scFv)₂-ETA' (SEQ ID NO: 8), respectively. Also the vectors comprising said nucleic acid molecules are embodiments of present invention.

Further embodiments are cells or non-human organisms synthesizing said complexes, obtainable by transforming or transfecting cells or non-human organism with the nucleic acid molecules or vectors of the above-described embodiments. The organisms or cells according to present invention are prokaryotes, such as *E. coli, B. subtilis, S. carnosus, S. coelicolor* or *Marinococcus sp.,* lower eukaryotes, such as *Saccharomyces sp., Aspergillus sp., Spodoptera sp.* or *P. pastoris.* Also an embodiment of present invention is a medicament containing the complex, nucleic acid molecules, vectors, cells/cellular extracts or single cell organism(s), such as prokaryotes and/or lower eukaryotes, or extracts of anyone of the organisms of the present invention. A further embodiment is the use of the complex of present invention, of the nucleic acid molecules and/or the vectors for influencing the cell growth and the physiology of CD64-positive cells; for that the cells are contacted with the complex and/or the nucleic acid molecules or vectors, optionally in the presence of transfection/transduction agents for proteins or nucleic acids, as they are well-known to the person skilled in the art. A further embodiment is the use of the complex, of the nucleic acid molecules coding therefore, of the vectors and/or of the cells or organisms for the preparation of a medicament for the treatment of acute myeloid leukemia, chronic inflammation reactions, such as: emphysema, intrinsic and extrinsic asthma, atopic dermatitis, polymorphic light eruption, SLE; graft versus host, multiple sclerosis, macrophage activation syndrome, rheumatoid arthritis, juvenile arthritis; Crohn's disease and chronic bowel disease. A further embodiment of the present invention is a method for influencing the cell growth and physiology of CD64⁺-cells comprising the following steps: (a) contacting cells, isolated tissue or isolated organs with the complex; or (b) transfecting cells isolated tissue or isolated organs with the nucleic acid molecules. Furthermore the present invention comprises a method to produce the recombinant complex of the invention comprising the following steps: transforming/transfecting cells, microorganism such as bacteria, fungi, and/or ciliates, with the nucleic acid molecules or vectors encoding the recombinant complex or components of it; (b) culturing the cells and/organisms of step (a) under conditions suitable for gene expression and protein synthesis in order to accumulate the recombinant complex intracellularly or in the surrounding culture medium; and (c) isolating and purifying the recombinant complex of step (b).

### Brief description of the drawings

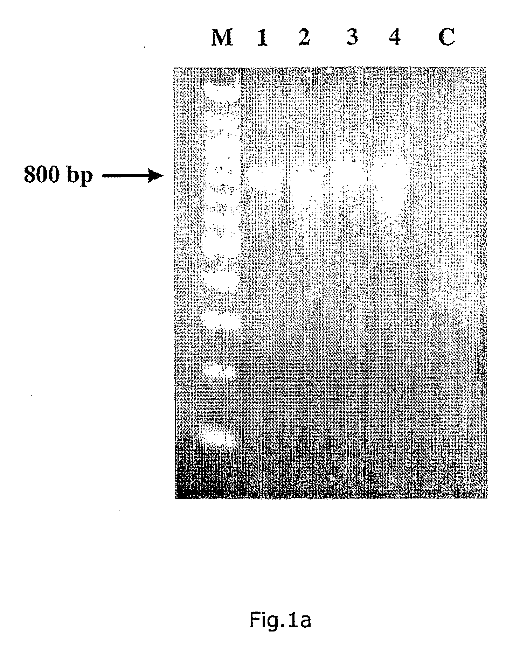

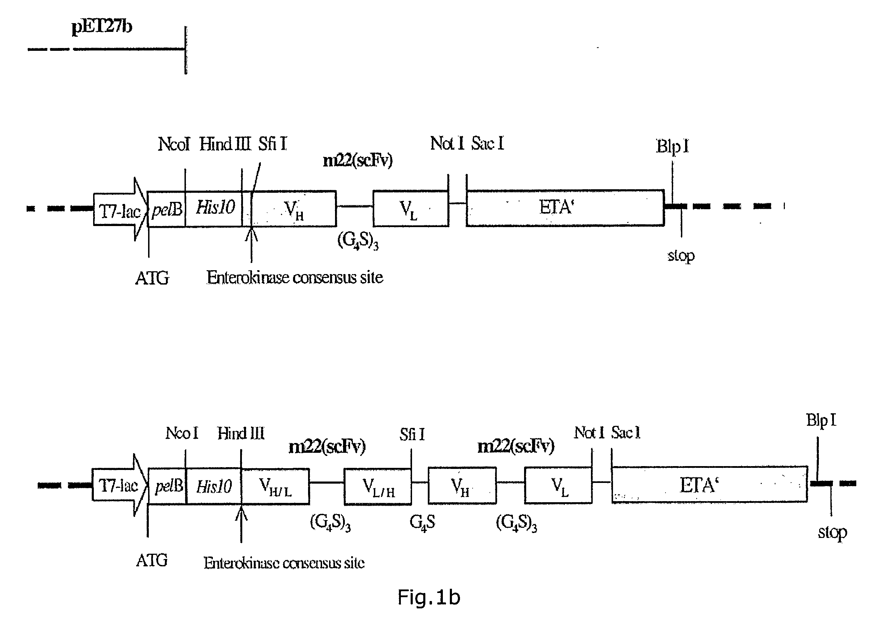

Figure 1: Cloning of m22(scFv)-ETA' and m22(scFV)₂-ETA'. (a) PCR amplification of m22(scFv) (M, 100 bp ladder; lane 1-4, m22(scFv) (852 bp); C, negative control) (b) Schematic structure of the monovalent m22(scFv)-ETA' and bivalent m22(scFV)₂-ETA' coding region in the pET27b *E. coli* expression vector. The expression module is composed of the IPTG inducible *T7-lac* operator, the signal peptide of the pectate lyase of *Erwinia carotovora-(peIB),* a synthetic and enterokinase-cleavable His₁₀ cluster *(His-Tag),* the anti-CD64 scFv m22 and the ETA' coding region.

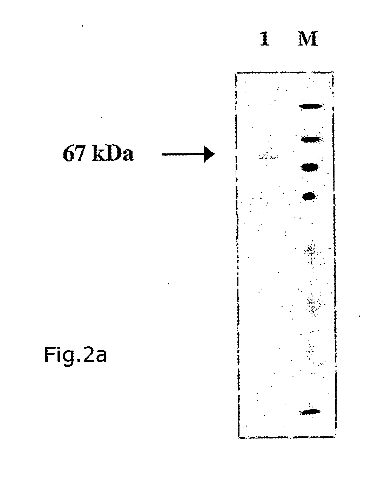

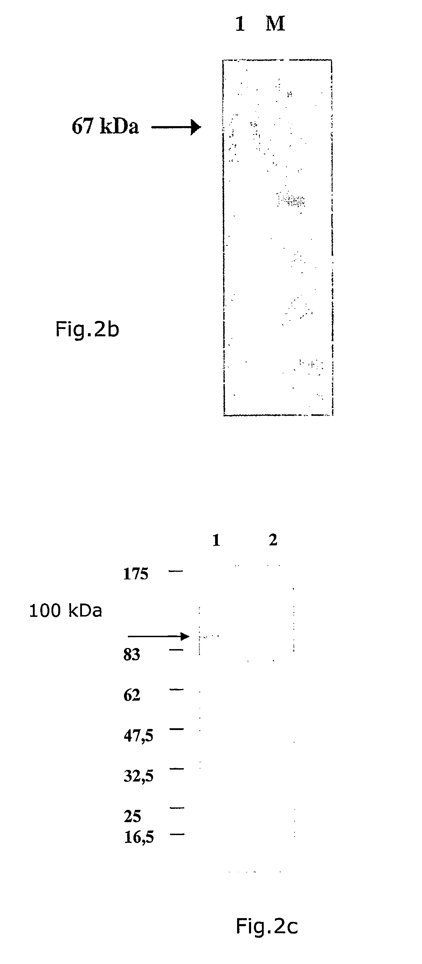

Figure 2: Bacterial expression and purification of m22(scFv)-ETA' and m22(scFv)₂-ETA' proteins; (a) m22(scFv)-ETA' protein after IMAC and SEC as documented after 10% (w/v) SDS-PAGE and Coomassie staining; 1, m22(scFv)-ETA' (67 kDa); M, Pre-stained Marker (Bio-Rad) (b) Immunoblot stained with anti-ETA' moAb TC-1 (lane identification corresponds to panel 2a); (c) m22(scFv)₂-ETA' protein after NiNTA and SEC as documented after SDS-PAGE, Westernblot and Comassie staining; M, Marker ; 1, m22(scFv)₂-ETA' (~ 100 kDa).

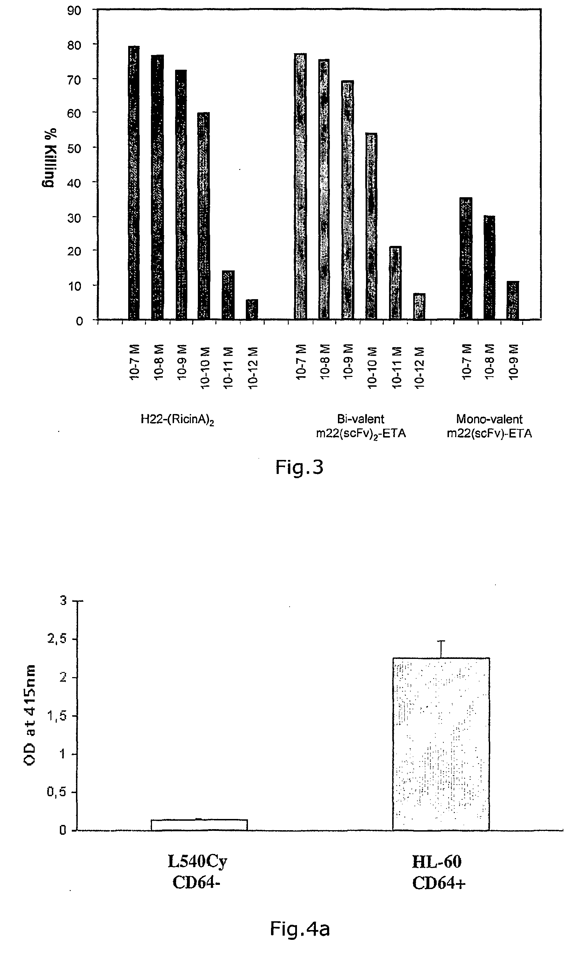

Figure 3: Efficacy comparison of CD64 immune toxins in *in vivo* killing of murine dermal inflammatory macrophages. Depicted are both the monovalent and bivalent recombinant toxin and the chemically linked whole H22 antibody to Ricin-A. The vertical axis shows the percentage killing compared to placebo injected sites and the horizontal axis contains the different Molar concentrations used referring to the toxin moiety.

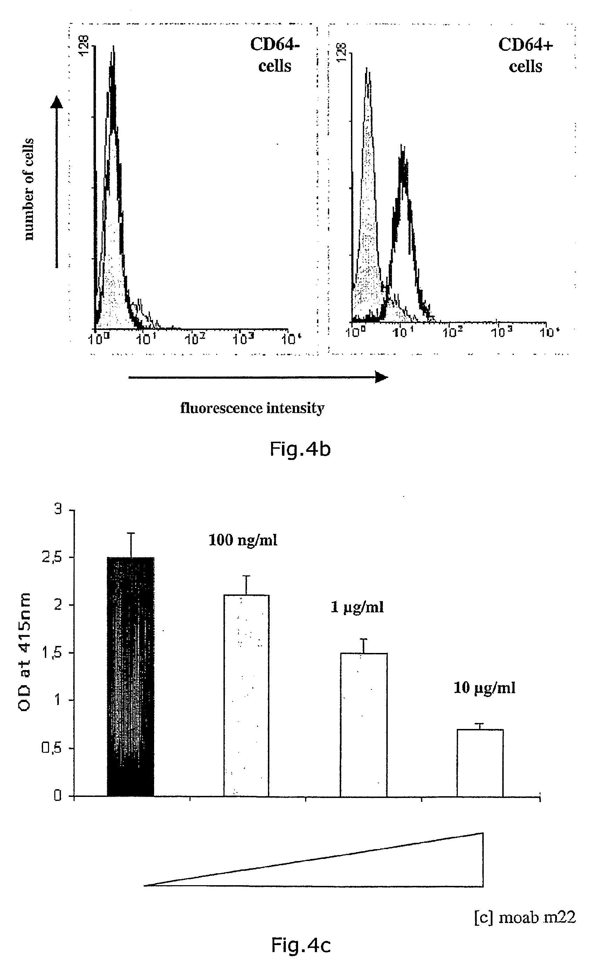

Figure 4: Binding properties of the recombinant anti-CD64 immunotoxin m22(scFv)-ETA'. (a) Binding of m22(scFv)-ETA' to CD64-negative cell membranes L540Cy and CD64-positive AML-derived cell membranes HL-60 documented by CM-ELISA. (b) Binding of m22(scFv)-ETA' to antigen-positive cells by flow or with PBS as negative control. A. L540Cy cells cytometry. Cells were stained with purified immunotoxin stained with CD64-specific immunotoxin. *B.* HL-60 cells stained with CD64-specific immunotoxin. (c) Documentation of specific binding activity of m22(scFv)-ETA' using a CM-ELISA with different dilutions of moAb m22 for competition. Binding of m22(scFv)-ETA' was detected with peroxidase-conjugated anti-His moAb. Presented are data from three independent experiments.

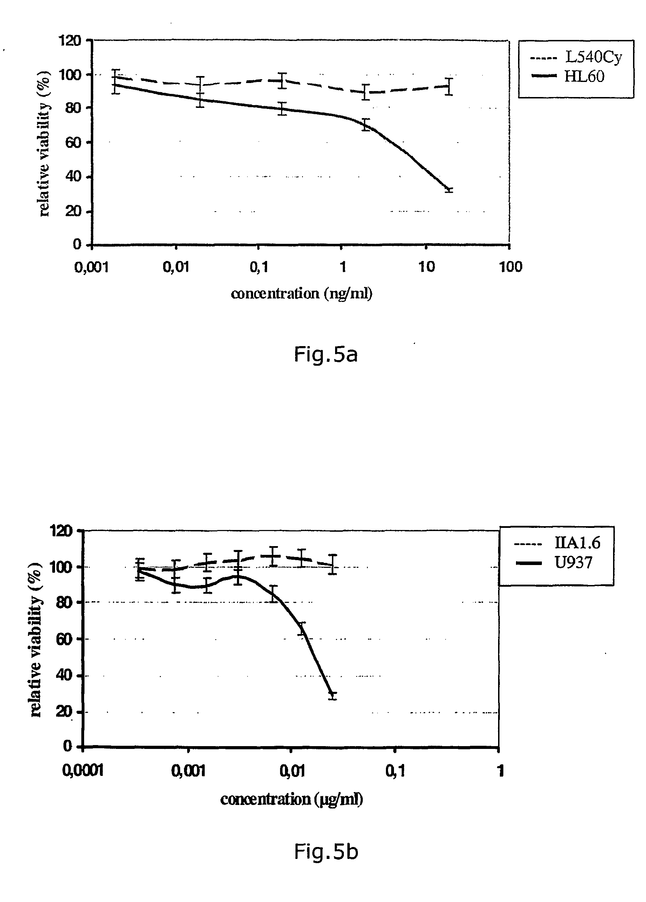

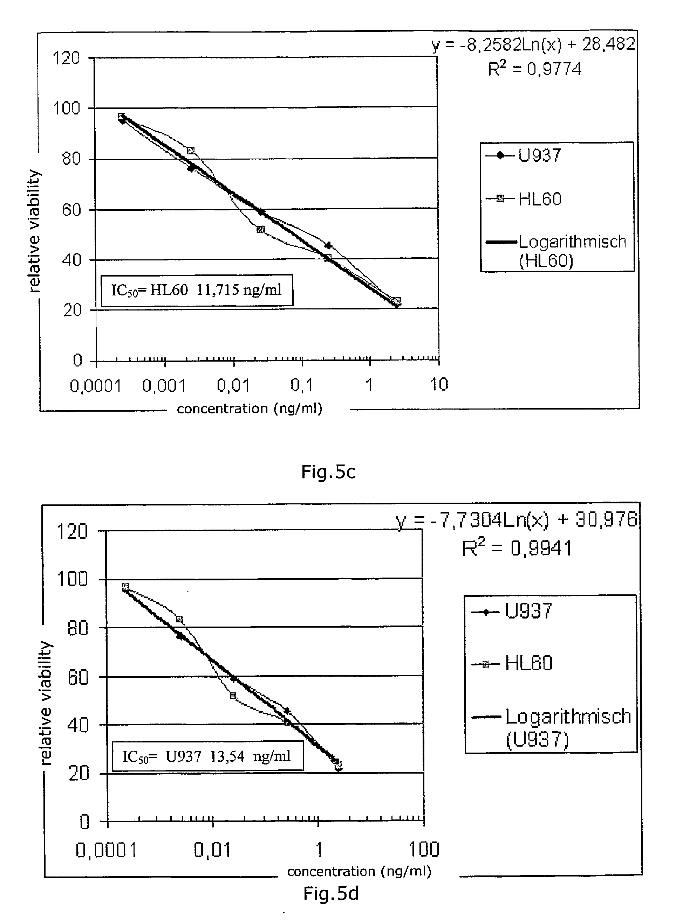

Figure 5: (a) Growth inhibition of AML-derived cell lines after incubation with the monovalent m22(scFv)-ETA' as documented by cell-viability assays (18). (a) HL-60 (CD64⁺) or L540Cy (CD64⁻) and (b) U937 (CD64⁺) or IIA1.6 (CD64⁻) were treated with various dilutions of recombinant anti-CD64 immunotoxin. HL-60, U937 (-) or L540Cy, IIA1.6 (---) were treated with m22(scFv)-ETA', and their ability to metabolize the XTT to a water-soluble formazan salt (formed by mitochondrial dehydrogenase activity) was measured as absorbance at 450 and 650 nm. Measurements were performed in triplicate. Results are presented as percentage of untreated control cells. (d) und (e) show the growth inhibition of myeloid cell line acute promyelocytic HL60 and histiocytic lymphoma cell-lines U 937 after incubation with the bivalent m22(scFv)₂-ETA' as documented by cell viability assays (18). In (d) and (e) HL60 (CD64⁺) or U937 (CD64⁺) were treated with rIT, and their ability to metabolize the XTT to a water-soluble formazan salt (formed by mitochondrial dehydrogenase activity) was measured as absorbance at 450 and 650 nm (reference wavelength) in a cell viability assay (18). Measurements were performed in triplicate. Results are presented as percentage of untreated control cells. Cytotoxic activity of m22(scFv)₂-ETA' (-) on HL60, U397 (----) cells and L540Cy (CD64⁻) cells were used as negative controls ( not showed, ~100 % viability). Measurements were again performed in triplicate. Display equation on chart U 937; y = -7,7304Ln(x) + 30,976; Display R-squared value on chart R2 = 0,9941; Display equation on chart HL 60; y = - 8,2582Ln(x) + 28,482; Display R-squared value on chart R2 = 0,9774; IC₅₀ m22(scFv)₂-ETA' = HL60 11,715 ng/ml; U937 13,54 ng/ml

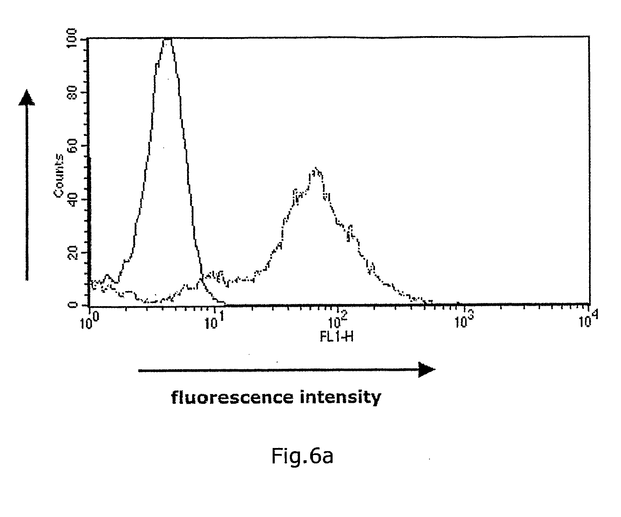

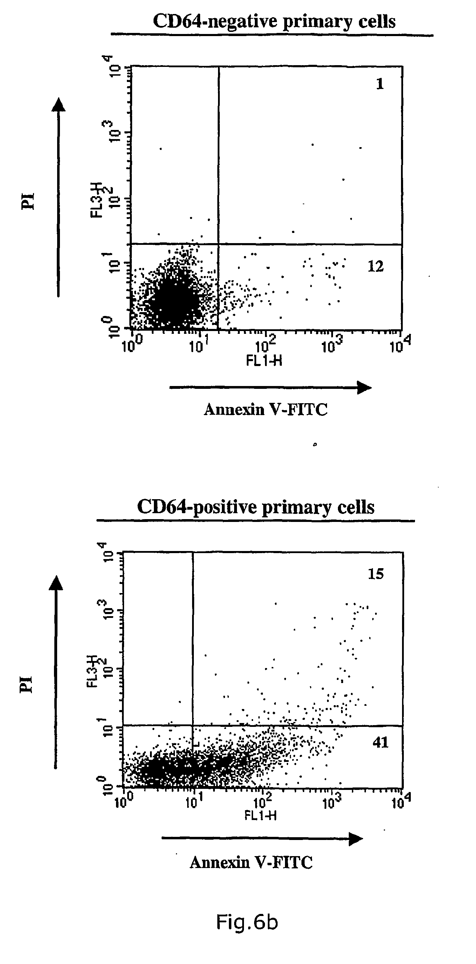

Figure 6: Flow cytometric analysis of induced apoptosis on fresh patient-derived AML cells by the recombinant anti-CD64 immunotoxin m22(scFv)-ETA'. (a) Binding of m22(scFv)-ETA' (transparent black lines) on primary cells. Cells were stained with Annexin-V-FITC and PI simultaneously. (b) Treatment of both, primary patient-derived CD64⁻- as well as CD64⁺-AML cells with 100 ng/ml of m22(scFv)-ETA'. Numbers in the lower and higher right quadrant of each plot represent the percentage of cells in early apoptosis (AnnV+/PI-) and late apoptosis/necrosis (AnnV+/PI+) respectively. The data are representative for three separate experiments performed with these samples.

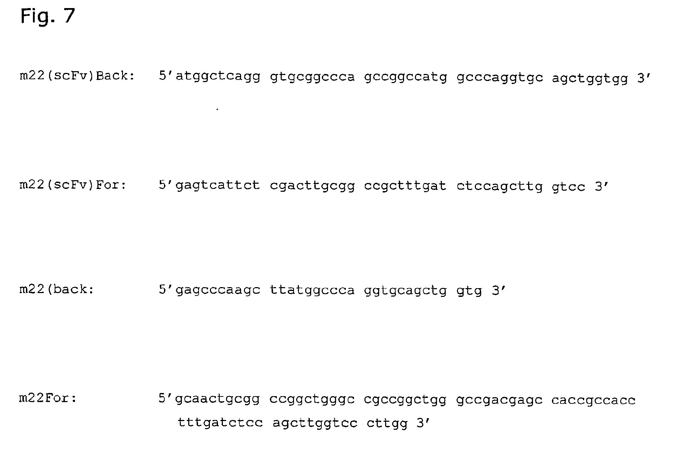

Figure 7: Primers used for the construction of the anti-CD64 immunotoxins.

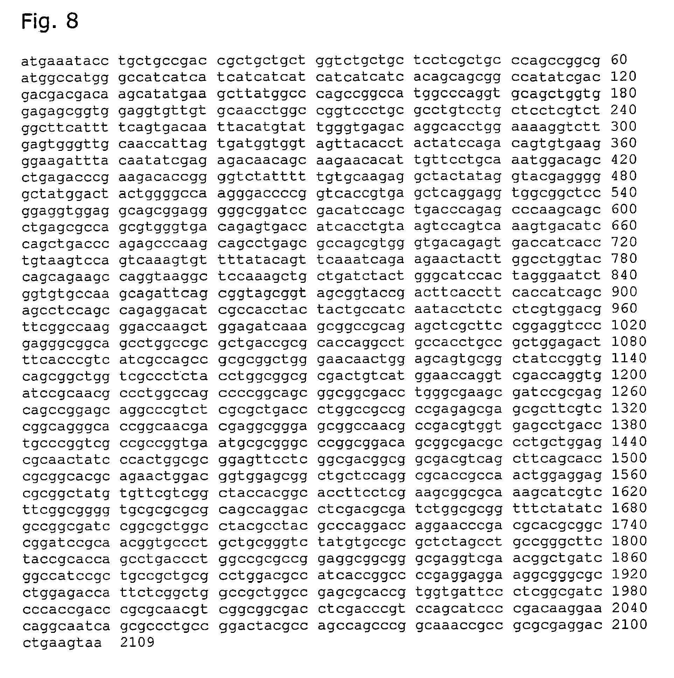

Figure 8: Nucleic acid sequence of the monovalent construct pBM-m22(scFv).

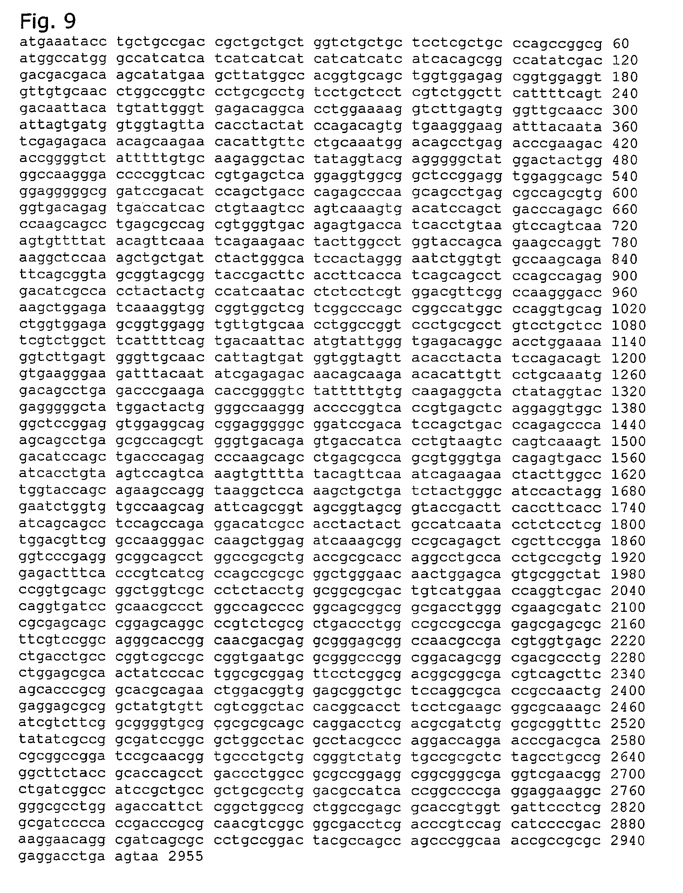

Figure 9: Nucleic acid sequence of the bivalent construct m22(scFv)₂-ETA'.

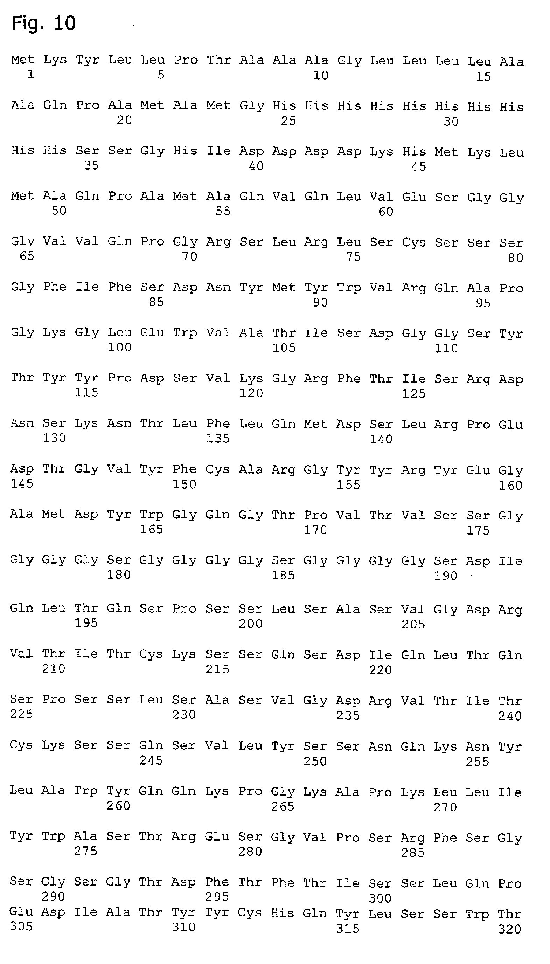

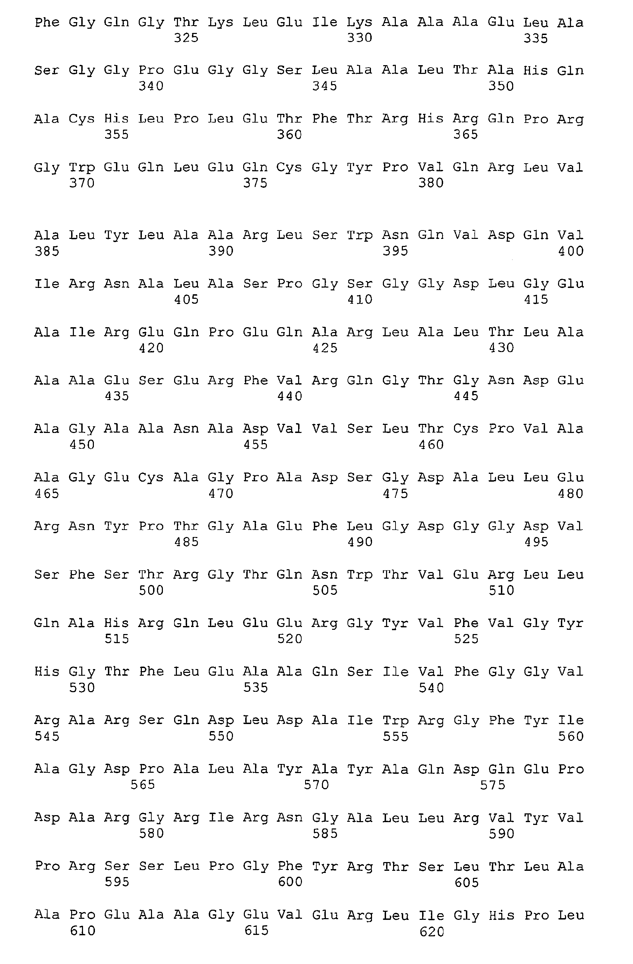

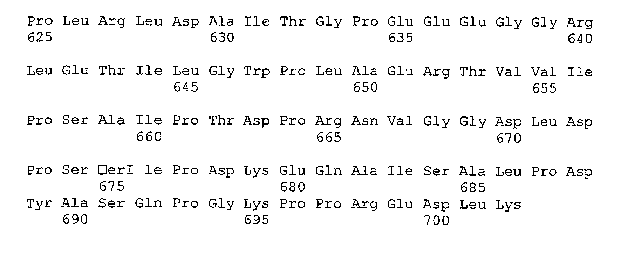

Figure 10: Amino acid sequence of the monovalent construct m22(scFv)-ETA'.

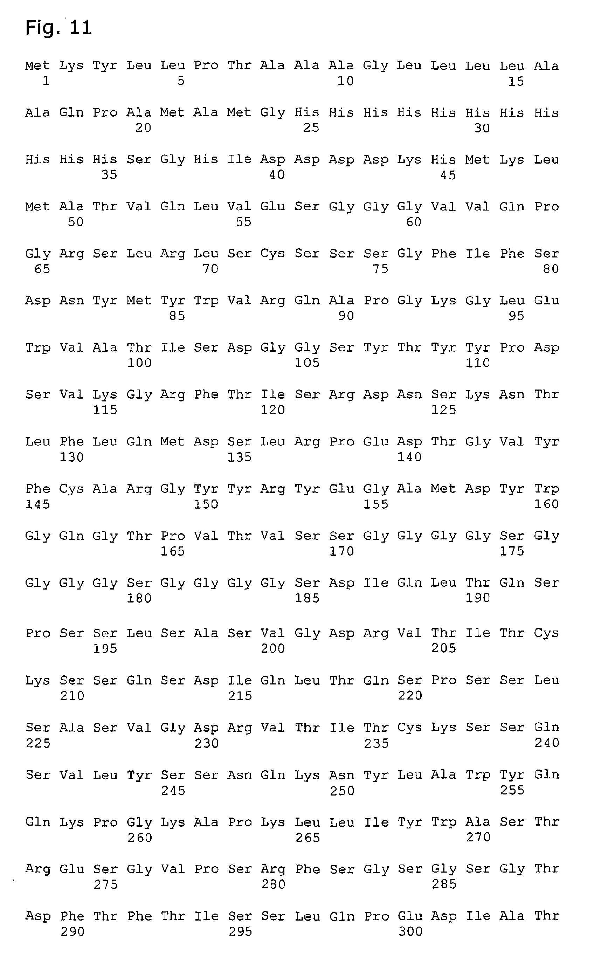

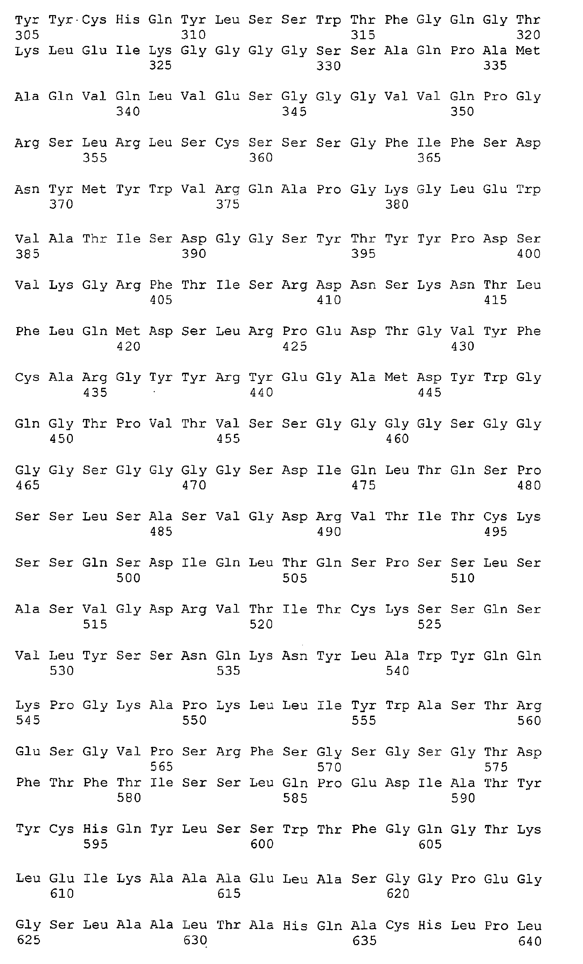

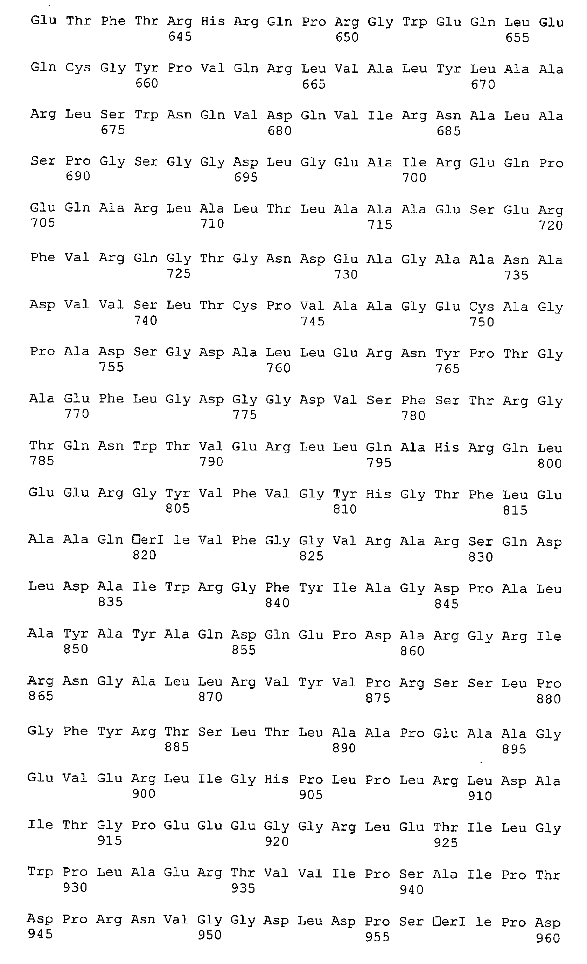

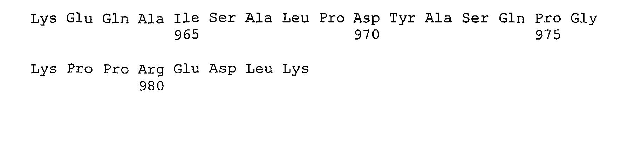

Figure 11: Amino acid sequence of the bivalent construct m22(scFv)₂-ETA'.

### Detailed description of the invention

The complex according to the invention are those mentioned in claim 1.

The scope of the present invention is defined by the claims and any information that does not fall within the claims is provided for information only.

### Definitions

As used herein, the term "complex" refers to linear molecules with different domains; said molecule may adopt a secondary and tertiary structure; the term "complex" also comprises a higher order non-linear structure made up of at least two molecules that only upon interaction form the active form of the complex. The interaction may result in a covalent bond between the at least two molecules and/or the at least two molecules are stabilised through non-covalent bonds. The interaction may occur *in vitro,* i.e. in a test tube, or other appropriate laboratory vessel, through chemically linking the at least two molecules or parts thereof and/or through the natural affinity of the at least two molecules or parts thereof for each other. The interaction may also occur *in vivo,* i.e. in a cell or in an organism, whereby for example cellular enzymes catalyse the formation of covalent bonds and/or non-covalent bonds.

As used herein, the term "immunotoxin" refers to chimeric molecules in which a cell-binding monoclonal antibody or fragments thereof are chemically coupled or genetically fused to toxins or their subunits. The toxin portion of the immunotoxin can be derived from various sources, such as bacteria, fungi, plants or animals. Toxins of human origin or synthetic toxins (drugs) can be used as well. Immunotoxins as well their construction have been reviewed above and are well known to the person skilled in the art.

As used herein, the term "component A" of the complex represents the CD64-binding domain of the complex of the present invention. combinations thereof; most preferred is a binding domain of higher valency generated by combining at least two of the binding molecules mentioned above.

As used herein, the term "antibody" refers to polyclonal antibodies, monoclonal antibodies, humanized antibodies, single-chain antibodies, and fragments thereof such as Fab, F(ab')2, Fv, and other fragments which retain the antigen binding function and specificity of the parent antibody.

As used herein, the term "monoclonal antibody" refers to an antibody composition having a homogeneous antibody population. The term is not limited regarding the species or source of the antibody, nor is limited by the manner in which it is made. The term encompasses whole immunoglobulins as well as fragments such as Fab, F(ab')2, Fv, and others, which retain the antigen binding function and specificity of the antibody. Monoclonal antibodies of any mammalian species can be used in this invention. In practice, however, the antibodies will typically be of rat or murine origin because of the availability of rat or murine cell lines for use in making the required hybrid cell lines or hybridomas to produce monoclonal antibodies.

As used herein, the term "human antibodies" means that the framework regions of an immunoglobulin are derived from human immunoglobulin sequences.

As used herein, the term "single chain antibody fragments" (scFv) refers to antibodies prepared by determining the binding domains (both heavy and light chains) of a binding antibody, and supplying a linking moiety, which permits preservation of the binding function. This forms, in essence, a radically abbreviated antibody, having only that part of the variable domain necessary for binding to the antigen. Determination and construction of single chain antibodies are described in U.S. Pat. No. 4,946,778 by Ladner et al.

The "toxin" moiety, here also called "cell-killing domain" of the complex is part of "component B" of the present invention and is Pseudomonas Exotoxin A.

As used herein, the terms "CD64" refer to a human surface molecule as extensively reviewed above. For immunization purposes CD64 antigen may be prepared by any technique known in the art.

Antibodies to human CD64 are known in the art. The present invention also contemplates a new use of recombinant derivatives of such antibodies as detailed above.

Alternatively, anti-human CD64 monoclonal antibodies can be produced using CD64 antigen as immunogen, provided that there is available a screening assay which distinguishes antibodies directed against other antigens present in the immunogenic composition. Also cells or membrane fractions containing the molecule of interest as immunogens may be used in order to preserve the conformational constraints provided by a membrane environment. Immunizing animals with whole cells or fractions thereof usually yields a strong immune response, which generates antibodies to a large number of different molecules. This broad immune response precludes the use of the CD64⁺- cells in combination with purified CD64 antigen in subsequent screening for specific antibody production by hybridoma clones derived from the mouse spleen or lymphocyte cells.

General techniques for raising polyclonal sera and monoclonal antibodies are set out below:

### a) Polyclonal Sera

Polyclonal sera may be prepared by conventional methods. In general, a solution containing the CD64 antigen is first used to immunize a suitable animal, preferably a mouse, rat, rabbit or goat. Rabbits and goats are preferred for the preparation of polyclonal sera due to the volume of serum obtainable, and the availability of labelled anti-rabbit and anti-goat antibodies. Immunization is generally performed by mixing or emulsifying the antigen-containing solution in saline, preferably in an adjuvant such as Freund's complete adjuvant, and injecting the mixture or emulsion parenterally (generally subcutaneously or intramuscularly). A dose of 50-200 µg/injection is typically sufficient. Immunization is generally boosted 2-6 weeks later with one or more injections of the protein in saline, preferably using Freund's incomplete adjuvant. One may alternatively generate antibodies by *in vitro* immunization using methods known in the art, which for the purposes of this invention is considered equivalent to *in vivo* immunization.

Polyclonal antisera are obtained by bleeding the immunized animal into a glass or plastic container, incubating the blood at 25°C for one hour, followed by incubating at 4°C for 2-18 hours. The serum is recovered by centrifugation (e.g., 1000 g for 10 minutes). About 20-50 ml per bleed may be obtained from rabbits.

### b) Monoclonal Antibodies

Monoclonal antibodies are prepared using the method of Kohler and Milstein, Nature (1975) 256:495-96, or a modification thereof. Typically, a mouse or rat is immunized as described above. However, rather than bleeding the animal to extract serum, the spleen (and optionally several large lymph nodes) are removed and dissociated into single cells. If desired, the spleen cells may be screened (after removal of non-specifically adherent cells) by applying a cell suspension to a plate or well coated with the protein antigen. B-cells expressing membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (e.g., hypoxanthine, aminopterin, thymidine medium, "HAT"). The resulting hybridomas are plated by limiting dilution, and are assayed for the production of antibodies, which bind specifically to the desired immunizing cell-surface antigen (and which do not bind to unrelated antigens). The selected mAb-secreting hybridomas are then cultured either *in vitro* (e.g., in tissue culture bottles or hollow fiber reactors), or *in vivo* (as ascites in mice).

As used in the present invention, the term "recombinant" refers to fusions of the antibody part to the toxin part by recombinant means such as through production of single chain antibodies, which are recombinantly expressed as part of a longer polypeptide chain, which also contains a toxin part. Recombinant immunotoxin produced in this way may be isolated by any technique known in the field of recombinant DNA expression technology suitable for this purpose.

As used herein, the term "vector" may comprise a plasmid, a cosmid, a phage, phagemid, derivatives of them or a virus. A "vector" may be used circular or linearized; a "vector" may be used as DNA or RNA.

As used herein, the term "organism" refers to prokaryotes and eukaryotes, single-cellular and multi-cellular ones. "Eukaryote" according to the present invention include lower eukaryotes, such as fungi, vertebrates such as mammals (human and non-human, including rodents etc.), birds and fish, invertebrates such as worms and insects, plants, etc. "Eukaryotic cell" according to the present invention relates to cells and cell culture derived from the above organisms, including stem cells.

In order to express the recombinant complex, the single chain antibody-toxin moiety fusion polypeptide (also termed "recombinant immunotoxin") of the present invention, in bacteria such as *E. coli,* or in yeast such as in S. *cerevisiae,* invertebrate hosts as insect cells, Chinese Hamster Ovary (CHO), COS, BHK, 293T and MDCK cells, the following steps are carried out:

Transformation or transfection of an appropriate cellular host with a recombinant vector, in which a nucleotide sequence coding for the fusion protein has been inserted under the control of the appropriate regulatory elements, particularly a promoter recognized by the polymerases of the cellular host and, in the case of a prokaryotic host, an appropriate ribosome binding site (RBS), enabling the expression in said cellular host of said nucleotide sequence.

As used herein, the expression "killing of CD64 expressing cells" is to be understood as implying an inhibition of protein synthesis or induction of apoptosis resulting in elimination or death of these cells; various molecular mechanism may be employed; for example mechanism that alter the function of a cell, those that alter the gene expression pattern of a cell or those that directly effect the viability of a cell may be used.

As used herein, the expression "CD64 expressing cells" refers to cells with CD64 as surface antigen. CD64 is mainly expressed on monocytes, macrophages and antigen presenting cells (APCs). Any type of cell expressing CD64 may be envisaged for treatment with the recombinant immunotoxins or compositions comprising the same of the present invention.

The invention also relates to nucleic acid molecules or vectors, which code for the complex according to the invention or for individual components for preparing the complex.

Also claimed are cells and/or organisms, which synthesize the complete complexes according to the invention or individual components thereof after being transformed or transfected with the nucleic acid molecules or vectors according to the invention.

The organisms according to the invention are either of prokaryotic origin, especially *E. coli, B. subtilis, S. carnosus, S. coelicolor, Marinococcus sp.,* or lower eukaryotes, especially *Saccharomyces sp., Aspergillus sp., Spodoptera sp., P. pastoris.*

The invention also relates to medicaments comprising a complex according to the invention; the invention further relates to medicaments comprising a nucleic acid coding for the said complex, or cells and/or cell extracts comprising the nucleic acid and/or said complex. Typically, the complexes, nucleic acids, cells or cellular extracts according to the invention are administered in physiologically acceptable dosage forms. These include, for example, Tris, NaCl, phosphate buffers and all approved buffer systems, especially including buffer systems, which are characterized by the addition of approved protein stabilizers. The administration is effected, in particular, by parenteral, intravenous, subcutaneous, intramuscular, intratumoural, transnasal administrations, and by transmucosal application.

The dosage of the complexes according to the invention to be administered must be established for each application in each disease to be newly treated by clinical phase I studies (dose-escalation studies).

Nucleic acids or vectors, which code for a complex according to the invention, are advantageously administered in physiologically acceptable dosage forms. These include, for example, Tris, NaCl, phosphate buffers and all approved buffer systems, especially including buffer systems which are characterized by the addition of approved stabilizers for the nucleic acids and/or vectors to be used. The administration is effected, in particular, by parenteral, intravenous, subcutaneous, intramuscular, intratumoural, transnasal administrations, and by transmucosal application.

The complex according to the invention, nucleic acid molecules coding for the complex or for components of it, cells and/or cellular extracts comprising the complex and/or nucleic acids coding for the complex or for components of it can be used for the preparation of a medicament for treating acute myeloid leukemia, and chronic inflammation reactions. In particular said medicament is useful for the treatment of chronic inflammation reactions, such as emphysema, intrinsic and extrinsic asthma; atopic dermatitis, polymorphic light eruption, SLE, graft versus host, multiple sclerosis, macrophage activation syndrome, rheumatoid arthritis, juvenile arthritis, Crohn's disease and chronic bowel disease, etc.

### Examples

### Methods

### Bacterial strains, oligonucleotides, and plasmids

*E.coli* XL1-blue (supE44 hsdR17 recA1 endA1 gyr A46 thi relA1 lacF'[pro AB+ lacI^{q} lacZΔM15 Tn10(tet^{r})]) was used for propagation of plasmids, and *E.coli* BL21 Star™ (DE3) (F ompT hsdSB(rB⁻mB⁻) gal dcm rne131 DE3) as host for synthesis of recombinant immunotoxins. Synthetic oligonucleotides were synthesized by MWG Biotech (Ebersberg, Germany). The bacterial expression vector pBM1.1 is derived from the pET27b plasmid (Novagen, Madison, USA), and is used for N-terminal fusion of Sfi I/Not I-ligands to the modified deletion mutant of *Pseudomonas aeruginosa* Exotoxin A (20). Plasmids were prepared by the alkaline lysis method and purified using plasmid preparation kits from Qiagen (Hilden, Germany). Restriction fragments or PCR products were separated by horizontal agarose gel electrophoresis and extracted with QIAquick (Qiagen). All standard cloning procedures were carried out as described by Sambrook *et al. (17).*

### Patient samples and cell lines

Heparinized peripheral blood samples from an adult patient with AML were obtained after informed consent and with the approval of the clinical research ethics board of the University of Aachen. Mononuclear cells (MNC) were isolated by low-density < 1.007 g/ml) gradient centrifugation using Ficoll-Paque PLUS™ (Amersham Biosciences, Freiburg, Germany) separation medium. All cell lines including the CD64⁺- AML-derived cell lines HL-60 (provided by T. Thepen, Utrecht, The Netherlands) and U937 (DSMZ; German Collection of Microorganisms and Cell Cultures, Braunschweig, Germany), and CD64⁻-L540Cy (16) and IIA1.6 (provided by T. Thepen), were cultivated in complete medium (RPMI 1640) supplemented with 10% (v/v) heat-inactivated fetal calf serum (FCS), 50 µg/ml penicillin, 100 µg/ml streptomycin and 2 mM L-glutamine. All cells were cultured at 37°C in a 5% CO₂ air atmosphere (16).

### Construction and expression of recombinant m22(scFv)-ETA' and m22(scFv)₂-ETA'

The m22(scFv) gene was amplified from m22-bearing plasmid (kindly provided by T. Thepen) by PCR using the oligonucleotide primer m22(scFv)Back: 5'-ATG-GCT-CAG-GGT-GC**G-GCC-CAG-CCG-GCC**-*ATG-GCC-CAG-GTG-CAG-CTG-GTG-G* (SEQ ID NO: 1); bold letters: Sfi I consensus site; in italics: 5'-m22(scFv) region; and the oligonucleotide primer m22(scFv)For: 5'-GAG-TCA- TTC- TCG-ACT -**TGC-GGC-CGC**-*TTT-GAT-CTC-CAG-CTT-GGT-CC* G (SEQ ID NO: 2); bold letters: Not I consensus site; in italics: 3'-m22(scFv) region. After Sfi I/Not I-digestion, the PCR-fragment was cloned into the bacterial expression vector pBM1.1 (16) and digested with the same restriction enzymes. The resulting monovalent (scFv) construct pBM-m22(scFv) (SEQ ID NO: 5) was verified by DNA sequence analysis. For construction of the bivalent m22(scFV)₂-ETA' immunotoxin (SEQ ID NO: 6), m22(scFv) was PCR-amplified with the primer m22(back): 5'- GAG-CCC-**AAG-CTT***-ATG-GCC-CAG-GTG-CAG-CTG-GTG* G (SEQ ID NO: 3); bold letters: HindIII consensus site; in italics: 5'-m22(scFv) region; and with the primer m22For: 5'-GCA-ACT-GC**G-GCC-GGC-TGG-GCC**-gcc-ggc-tgg-gcc-gac-gag-cca-ccq-cca-cc*T-TTG-ATC-TCC-AGC-TTG-GTC-CCT-TG* G (SEQ ID NO: 4); bold letters: Sfi I consensus site; underlined letters: linker region; in italics: 5'- m22(scFv) region. After Hindlll/Sfil-digestion, the PCR fragment was cloned into pBM-m22(scFv), digested with the same restriction sites, to get the bivalent construct m22(scFv)₂-ETA' (SEQ ID NO: 6). After transformation into BL21 Star^{™} (DE3), the m22(scFv)-ETA' (SEQ ID NO: 7), and m22(scFv)₂-ETA' (SEQ ID NO: 8) fusion proteins were periplasmically expressed under osmotic stress in the presence of compatible solutes, as described by Barth *et al,* (16). Briefly (the following section entitled "Periplasmatic expression and purification of the recombinant m22(scFv)₂-ETA'" provides a more detailed description of the method), transformed bacteria were harvested 15 h after IPTG induction. The bacterial pellet was resuspended in sonication-buffer (75 mM Tris/HCl (pH 8), 300 mM NaCl, 1 capsule of protease inhibitors/50 ml (Complete^{™}, Roche Diagnostics, Mannheim, Germany), 5 mM DTT, 10 mM EDTA, 10% (v/v) glycerol) at 4°C and sonicated 6 times for 30 s at 200 W. The m22(scFv)-ETA' fusion proteins (SEQ ID NO: 7), were enriched by IMAC (immobilized metal-ion affinity chromatography) using nickel-nitriloacetic chelating Sepharose (Qiagen) and SEC (size exclusion chromatography) with Bio-Prep SE-100/17 (Biorad, München, Germany) columns according to the manufacturer's instructions. Recombinant Protein was eluted with PBS (pH 7.4) and 1 M NaCl, analyzed by Sodium dodecyl sulphate/polyacrylamide gel electrophoresis (SDS-PAGE), quantified by densitometry (GS-700 Imaging Densitometer; Biorad) after Coomassie staining in comparison with BSA standards (figures 2a and 2b) and verified by Bradford assays (Biorad).

### Periplasmatic expression and purification of the recombinant m22(scFv)₂-ETA'

Periplasmic Expression and Purification of the Recombinant m22(scFv)₂-ETA'. Recombinant Its (rIT) were expressed under the control of the IPTG inducible T7lac promoter in E.coli BL21 (DE3) as described recently (1). Briefly, bacteria were grown overnight at 26°C in Terrific Broth (30) containing 50 µg/ml kanamycin and 0.5 mM ZnCl₂. The culture was diluted 30-fold in 200 ml of the same medium. At an A₆₀₀ of 2 it was supplemented with 0.5 M sorbitol, 4% NaCl, and 10 mM glycine betaine, and incubated at 26°C for and additional 30-60 min. Expression of rIT was induced by the addition of 2 mM IPTG at 26°C. Later (15 h), cells were harvested by centrifugation at 3,700 g for 10 min at 4°C. For all of the next steps, tubes were kept on ice. The bacterial pellet was centrifuged and its wet weight determined. Cells were frozen at - 196°C. After thawing, the cells were resuspended in 75 mM Tris-HCl (pH 8), 300 mM NaCl, 1 capsule of protease inhibitors/50 ml (Complet, Roche Diagnostics, Mannheim, Germany), 5 mM DTT, 10 mM EDTA, and 10% (v/v) glycerol, and sonicated six 6 times for 30 s at 200 W. The periplasmic fraction was recovered after centrifugation at 21,000 g for 5 min at 4°C and transferred to PBS (pH 8), 300 mM NaCl, and 10% glycerol using PD10 desalting columns (Pharmacia, Freiburg, Germany). rIT was partially purified by immobilized metal-ion affinity chromatography that contained nickel-nitriloacetic acid chelating Sepharose (Qiagen) on a Äkta FPLC (Pharmacia Biotech, Uppsala, Sweden). Bound protein was eluted with 500 mM imidazole PBS (pH 8), 1 M NaCl, and 10% glycerol. Fractions containing the bivalent m22(scFv)₂-ETA' were pooled, concentrated by ultrafiltration, and finally purified using SEC with HI-Prep 26/60 Sephacryl S100 columns (Pharmacia Biotech, Uppsala, Sweden) on the Äkta FPLC workstation. Protein was eluted with PBS (pH 7.4), analyzed by SDS-PAGE, quantified after Coomassie staining in comparison with BSA standards (figure 2 c) and verified by Bradford assays (Bio-Rad) .

### SDS-PAGE and Western Blot Analysis

SDS-PAGE and Western blotting were performed as described (16). m22(scFv)-ETA' (SEQ ID NO: 7) was detected by anti-ETA' moAb TC-1 (20) (kindly provided by O.R. Galloway, Columbus, Ohio). Bound antibody was stained with an alkaline-phosphatase-conjugated anti-mouse-IgG moAb (Sigma Chemical Co., Deisenhofen, Germany) and a solution of Tris-HCl (pH 8.0) and 0.2 mg/ml naphtol-AS-Bi-phosphate (Sigma Chemical Co.) plus 1 mg/ml Fast-Red (Serva, Heidelberg, Germany).

### Cell membrane (CM) ELISA

The binding activity of the fusion proteins m22(scFv)-ETA' (SEQ ID NO: 7) and m22(scFv)₂-ETA' (SEQ ID NO: 8) were determined by CM-ELISA using biological active membranes of tumour cells as described by Tur *et al. (19)* (ELISA Maxisorp-Plates (Nalgen Nunc International, Roskilde, Denmark) were coated with 100 µl (approximately 0.9 mg protein/ml) freshly prepared membrane fractions of CD64⁺-cell lines HL-60/U-937 and L-540Cy as control in 0.02 M bicarbonate buffer (pH 9.6) overnight at 4°C. Plates were washed five times with PBS (pH 7.4) containing 0.2% Tween 20 (TPBS) and blocked with 200 µl 2% BSA in PBS (PBSA). After overnight incubation at 4°C, plates were washed five times with TPBS and 2-10 µg/ml of m22(scFv)-ETA' (SEQ ID NO: 7) diluted with 0.5% BSA and 0.05% Tween 20 in PBS was added to the plates and incubated at room temperature (23°C) RT for 1h. Thereafter, plates were washed and binding of the recombinant immunotoxin was detected with the anti-ETA' moAb TC-1 and F(ab')2 fragments of peroxidase-coupled goat anti-mouse IgG (Boehringer, Ingelheim, Germany) according to the manufacturer's recommendations. Bound antibodies were visualized after addition of 100 µl 2', 2'-azino-bis(3-ethylbenzthiazoline-6-sulphonic acid) (ABTS^{®}) solution (Roche Molecular Biochemical's, Mannheim, Germany) by measuring the extinction at 415 nm with an ELISA-Reader (MWG Biotech).

### Binding Specificity

The binding specificity of the recombinant immunotoxins were tested by CM-ELISA following the protocol described above using the parenteral moAb m22 for competition (Acris, Bad Nauheim, Germany). Briefly, ELISA Maxisorp plates were coated with membrane fractions of CD64-positive HL-60 cells. After blocking, plates were incubated with a fixed concentration (~35 µg/ml) of recombinant immunotoxin m22(scFv)-ETA' (SEQ ID NO: 7). Competition experiments were performed in the presence or absence of different concentrations (100 ng - 10 µg/ml) of moAb m22. Binding of m22(scFv)-ETA' (SEQ ID NO: 7) was detected using anti-His-Peroxidase (Roche Molecular Biochemical's) after addition of ABTS.

### Flow cytometry analyses

Cell binding activity of m22(scFv)-ETA' (SEQ ID NO: 7) and m22(scFv)₂- ETA' (SEQ ID NO: 8) expressed in E. *coli* BL21 Star^{™} (DE3) were evaluated using a FACSCalibur flow cytometry instrument and CellQuest software (Becton Dickinson, Heidelberg, Germany). Cells were stained with scFv-immunotoxin as described (25). Briefly, ten thousand events were collected for each sample, and analyses of intact cells were performed using appropriate scatter gates to exclude cellular debris and aggregates. 5 x 10⁵ cells were incubated for 1 h on ice with 50 µl of the m22(scFv)-ETA' (SEQ ID NO: 7) bacterial protein extract at a concentration of 30-40 µg/ml. The cells were washed with PBA buffer containing 0.2% w/v BSA and 0.05% w/v sodium azide and then incubated for 30 min with anti-TC-1 moAb diluted 1:2 in PBA buffer. Cells were washed and incubated with fluorescein-iso-thiocyanate (FITC)-labeled goat-anti-mouse IgG (DAKO Diagnostica, Hamburg, Germany) for 1 h at 4°C. After a final wash, the cells were treated with 2 µl 6,25 mg/ml propidiumiodide and subsequently analyzed by FACS.

### Colorimetric cell proliferation assay

The cytotoxic effect of m22(scFv)-ETA' (SEQ ID NO: 7) on target cell lines was determined by measurement of metabolization of yellow tetrazolium salt (XTT) to a water soluble orange formazan dye was determined as published by (25). Various dilutions of the recombinant toxin were distributed in 100 µl aliquots in 96-well plates. 2-4 × 10⁴ target cells in 100 µl aliquots of complete medium were added and the plates were incubated for 48 h at 37°C. Afterwards, the cell cultures were pulsed with 100 µl fresh culture medium supplemented with XTT/PMS (final concentrations of 0.3 mg and 0.383 ng respectively) for 4 h. The spectrophotometrical absorbances of the samples were measured at 450 and 650 nm (reference wavelength) with an ELISA reader (MWG Biotech). The concentration required to achieve a 50% reduction of protein synthesis (IC₅₀) relative to untreated control cells and to 1% Triton X treated positive controls was calculated graphically via Excel generated diagrams. All measurements were done in triplicate.

### Flow cytometric assay of apoptosis

Mononuclear cells from patient-blood samples were isolated by Ficoll-Paque centrifugation. Cell-surface CD64-expression was confirmed by flow cytometry using the m22(scFv)-ETA' immunotoxin as described above. Approximately 5 x 10⁵ mononuclear cells/well were seeded in flat-bottomed 12-well plates in RPMI 1640 supplemented with 10% FCS in triplicate. 100 ng/ml immunotoxin were added into each well, and the cells cultured for 18 h at 37°C and 5% CO₂ air atmosphere. Apoptotic cells were detected using an annexin V-FITC apoptosis detection kit I (BD Pharmingen, Heidelberg, Germany). Briefly, whole cells were stained simultaneously with FITC-conjugated Annexin V (AnnV) and PI in PBS according to the manufacturer's protocol. Ten thousand cells were analyzed by flow cytometry, and the AnnV-/PI-, AnnV+/PI-, AnnV+/PI+ and AnnV-/PI+ subpopulations were counted. Early apoptotic cells with exposed phosphatidylserine but intact cell membranes bound to Annexin V-FITC but excluded PI. Thus, the four populations of AnnV-/PI-, AnnV+/PI-, AnnV+/PI+ and AnnV-/PI+ have been found to correspond to living cells, early apoptotic cells, late apoptotic/necrotic cells and necrotic cells, respectively.

### Induction of cutaneous inflammation, immunotoxin injections, and biopsies

In all experiments, transgenic FVB/N mice expressing human FcγRI were used. To induce chronic cutaneous inflammation, an area of 1.5 by 1.0 cm on both flanks of the mice was shaved off and the irritant sodium lauryl sulfate (SLS) (5% in saline) was daily applied epicutaneously on ten consecutive days. Animals were anaesthetized with 20 µl of a 4:3 mixture of Aescoket (Aesculaap, Gent, Belgium) and Rompun (Bayer, Leverkusen, Germany), intramuscularly injected. Intradermal injections, (10 µl; 10:1 in saline; the M range in figure 3 refers to the toxin moiety) with the test samples in different concentrations were administered. For control purposes, identical saline injections were administered contralaterally. Animals were killed and 3 mm punch biopsies were taken, snap frozen in liquid nitrogen and stored at -70°C prior to use.

### Immunohistochemical CD64-staining

Biopsies were cut into 6 µm sections on a freezing microtome and mounted on coated slides. After drying overnight, the sections were fixed for 10 min with dry acetone and air-dried. Slides were incubated with FITC conjugated 10.1 (Serotec 1:40) in PBS 2% normal mouse serum (NMS) for 45 min. Slides were washed three times for 5 min with PBS, 0.05% Tween, after which alkaline phosphatase (AP) conjugated sheep anti FITC (Boehringer Mannheim, 1:400) in PBS (1% Human AB serum, 1% NMS for 30 min). After washing twice in PBS/Tween and once in Tris-HCI (0.1 M, pH 8.5), AP activity was demonstrated using naphtol AS-BI phosphate (sodium salt, 50 mg/100ml; Sigma) as substrate and new fuchsin (10 mg/100ml; Merck, Whitehouse Station, N.J.) as chromogen dissolved in 0.1 M Tris-HCI, pH 8.5, resulting in pink/red staining. Endogenous AP activity was inhibited by addition of levamisole (35 mg/100ml, Sigma) to the reaction mixture. Slides were lightly counterstained with hematoxylin. Sections were scored for positive cells by two independent observers and percentage of killing was calculated compared to non-injected sites.

### Results

### Construction and expression of m22(scFv)-ETA' and m22(scFv)₂-ETA'

PCR-amplified m22(scFv) (figure 1a) and m22(scFv)₂ were directionally inserted into kanamycin-resistant pBM1.1 containing an IPTG-inducible *lac* operator, a *pe*/*B* signal peptide followed by an enterokinase-cleavable His₁₀ tag, and modified ETA' (figure 1b). The deleted domain Ia of *Pseudomonas* Exotoxin responsible for non-specific cell recognition was thus replaced by CD64-specific m22(scFv) or m22(scFv)₂'. Successful cloning was verified by sequence analysis.

After transformation, *E.coli* BL21 Star^{™} (DE3) were cultivated under osmotic stress conditions in the presence of compatible solutes. The recombinant immunotoxin was directed into the periplasmic space and the functional m22(scFv)-ETA' (SEQ ID NO: 7) (Mᵣ 67,000) or m22(scFv)₂-ETA' (SEQ ID NO: 8) (Mᵣ 106,000) directly purified by combinations of IMAC and SEC to >90% purity. At least 1 mg of purified protein was routinely prepared from 1 l of bacterial shaking cultures (figure 2a and 2c). Intact recombinant immunotoxin was secreted to the periplasmic compartment, as visualized by immunoblot using TC-1, an ETA'-specific monoclonal antibody (figure 2b).

### Binding properties of CD64-specific Immunotoxin

Fusion of the scFv coding regions to the truncated ETA' coding sequences did not affect the binding activity of the VH/VL antibody format. Purified recombinant anti-CD64 immunotoxin always bound to AML cell membrane fractions but not to CD64-negative L540Cy membranes and corresponding intact cells as measured by CM-ELISA (figure 4a) and flow cytometry (figure 3b), respectively. CD64-specificity was documented by competitive CM-ELISA experiments: binding of m22(scFv)-ETA' against CD64-positive HL-60 membrane fractions was inhibited ~70% by 10 µg/ml moAb m22 (figure 4c).

### In vitro cytotoxic activity

To characterize the cytotoxic activity of the recombinant anti-CD64 immunotoxin *in vitro,* we evaluated the proliferation of different target cells after incubation with different amounts of m22(scFv)-ETA' (SEQ ID NO: 7). Growth inhibition of AML-derived cell lines HL-60 and U937 were documented by a XTT -based colorimetric assay. Toxic effects were observed against CD64⁺-cells with a calculated median IC₅₀ of 11.6 ng/ml on HL-60 cells (figure 5a) and 12.9 ng/ml on U937 cells, respectively (figure 5b). The CD64-negative Hodgkin-derived cell line L540Cy was not affected by recombinant protein concentrations of up to 10 µg/ml. Analogous experiments were performed with the bivalent protein m22(scFv)₂-ETA (figure 5d and 5e). The IC₅₀ on HL-60 cells was determined to be 11.715 ng/ml and on U937 cells, 13.54 ng/ml.

### Analysis of apoptosis on primary AML cells

The effects of m22(scFv)-ETA' on the induction of apoptosis in a freshly prepared population of acute myeloma cells from a patient were examined by flow cytometry. Immunophenotyping revealed ~90% leukemic cells. The expression of CD64 on the primary cells was verified directly with m22(scFv)-GFP and in a sandwich approach with m22(scFv)-ETA' (figure 5a). Two colour flow cytometric analysis using Annexin-V-FITC and PI (figure 5b) discriminated four populations, viable (lower left quadrant), early apoptotic (lower right quadrant), late apoptotic/necrotic (upper right quadrant) and necrotic cells (upper left quadrant). Primary patient-derived CD64-negative leukemic-cells treated with m22(scFv)-ETA' for 18 h remained mostly viable (~90%). Primary patient-derived CD64⁺-AML-cells treated with the recombinant immunotoxin after incubation for 18 h showed viable (~44%), early apoptotic (~41%) and late apoptotic/necrotic cell populations (~15%).

### In vivo cytotoxic activity

To establish *in vivo* efficacy of the recombinant anti-CD64 immunotoxins the ability to eliminate activated macrophages from the skin in a hCD64 transgenic mouse model was tested. Chronic inflammation was induced through epicutaneous application of the irritant sodium lauryl sulfate (5% in PBS), resulting in local chronic cutaneous inflammation. Test samples were injected intracutaneously and efficacy was determined by immunohistochemical staining for CD64 expressing cells in biopsies, taken from treated cutaneous sites after 24 hours and comparison with placebo treated sites. Two recombinant anti-CD64 immunotoxins, mono-valent (m22(scFv)-ETA') (SEQ ID NO: 7), and bivalent (m22(scFV)₂-ETA') (SEQ ID NO: 8), were tested and efficacy, expressed as percentage killing of CD64-expressing cells compared to placebo, was compared to a Ricin-A-based chemically-linked toxin, (H22-Ricin A), and a chemically-linked ETA toxin, (M22-ETA), table 1. Bivalent recombinant anti- CD64 immunotoxin showed a significant increase in killing CD64 expressing cells *in vivo,* in comparison to the monovalent version, and with a similar dose response curve compared to chemically-linked immunotoxins.

**Table 1: Efficacy as % killing of CD64 expressing cells compared to placebo.**

| Concentration (M) Toxin | 10⁻⁷ | 10⁻⁸ | 10⁻⁹ |
|---|---|---|---|
| H22-Ricin A | 99 | 91 | 79 |
| M22-ETA | 90 | 82 | 68 |
| m22(scFv)₂-ETA' | 97 | 88 | 74 |
| m22(scFv)-ETA' | 36 | 30 | 10 |

### References

1. Kaminski, M.S. , Zasadny, K.R., Francis, I.R., Fenner, M.C., Ross, C.W., Milik, A.W., Estes, J., Tuck, M., Regan, D., Fisher, S., Glenn, S.D., and Wahl, R.L. Iodine-131-anti-B1 radioimmunotherapy for B-cell lymphoma. J. Clin. Oncol. 14: 1974-1981, 1996.
2. Pennel, C.A. and Erickson, H.A. Designing immunotoxins for cancer therapy. Immunol Res. 25: 177-191, 2002.
3. Chaudhary, V.K., Gallo, M.G., FitzGerald, D.J. and Pastan, I.A. Recombinant single chain immunotoxin composed of anti-Tac variable regions and a truncated diphtheria toxin. Proc. Natl. Acad. Sci. USA 87: 9491-9494, 1990.
4. Brinkmann, U., Keppler- Hafkemeyer, A. and Hafkemeyer, P. Recombinant immunotoxins for cancer therapy. Expert Opin. Biol. Ther. 1: 693-702, 2001.
5. Frankel, A.E., Tagge, E.P. and Willingham, M.C. Clinical trials of targeted toxins. Semin. Cancer Biol. 6: 307-317, 1995.
6. Youle, R.J., Newton, D., Wu, Y.N., Gadina, M. and Rybak, S.M. Cytotoxic ribonucleases and chimeras in cancer therapy. Crit. Rev. Ther. Drug carrier Syst 10: 1-28, 1993.
7. Fett, J.W., Strydom, D.J., Lobb, R.R., Aldermann, E.M., Bethune, J.L., Riordan, J.F. and Vallee, B.L. isolation and characterization of angiogenin, an angiogenic protein from human carcinoma cells. Biochemistry 24: 5480-5486, 1985.
8. Huhn, M., Sasse, S., Tur, M.K., Matthey, B., schinkothe, T., Rybak, S.M., Barth, S. and Engert, A. Human angiogenin fused to human CD30 ligand (Ang-CD30L) exhibits specific cytotoxicity against CD30-positive lymphoma. Cancer Res. 61: 8737-8742, 2001.
9. Newton, D.L. and Rybak, S.M. Preparation and preclinical characterization of of RNase-based immunofusion proteins. Methods Mol. Biol. 160: 387-406, 2001.
10. Fossati, G., Bucknall, R.C. and Edwards, S.W. Fcgamma receptors in autoimmune diseases. Eur. J. Clin. Invest. 31: 821-831, 2001.
11. Menendez, P., del Canizo, M.C. and Orfao, A. Immunophenotypic characteristics of PB-mobilised CD34+ hematopoietic progenitor cells. J. Biol. Regul. Homeost. Af'gents 15: 53-61, 2001.
12. Liu Yin, J.A., Minimal residual disease in acute myeloid leukaemia. Best Pract. Res. Clin. Haematol. 15: 119-135, 2002.
13. Carter, P. Improving the efficacy of antibody-based cancer therapies. Nat. Rev. Cancer 1: 118-129, 2001.
14. Zhong, R.K., van de Winkel, J.G., Thepen, T., Schultz, L.D. and Ball, E.D. Cytotoxicity of anti-CD64-ricin a chain immunotoxin against human acute myeloid leukaemia cells in vitro and in SCID mice. J. Hemather. Stem Cell Res. 10: 95-105, 2001.
15. Bera, T.K., Williams-Gould, J., Beers, R., Chowdhury, P. and Pastan, I. Bivalent disulfide-stabilized fragment variable immunotoxin directed against mesotheliomas and ovarian cancer. Mol. Cancer Ther. 1: 79-84, 2001.
16. 16Barth S., Huhn M., Wels W., Diehl V., Engert A. Construction and /in vitro/ evaluation of RFT5(scFv)-ETA', a new recombinant single-chain immunotoxin with specific cytotoxicity toward CD25+ Hodgkin-derived cell lines. Int. J. Molec. Med. 1: 249-256 (1998).
17. Sambrook, J., Fritsch, E., and Maniatis, T. (eds.). Molecular Cloning. A laboratory manual.
18. Jost, L. M., Kirkwood, J. M., and Whiteside, T. L. Improved short- and long-term XTT-based colorimetric cellular cytotoxicity assay for melanoma and other tumor cells. J. Immunol. Methods, 147: 153-165, 1992.
19. Tur M.K., Huhn M., Sasse S., Engert A., Barth S. - Selection of scFv phages on intact cells under low pH conditions leads to a significant loss of insert-free phages. BioTechniques 30 (2): 404-413 (2001).
20. McGowan J.L., Kessler S.P., Anderson D.C., Galloway D.R. Immunochemical analysis of Pseudomonas aeruginosa exotoxin A. Analysis of the His426 determinant. J. Biol. Chem. 266(8): 4911-4916 (1991).

## Claims

1. A recombinant complex having at least one component A, at least one component B, whereby component A comprises two or more binding domains for the cellular surface receptor CD64, and component B is a cell-killing domain wherein the complex is the monovalent recombinant complex m22(scFv)₂-ETA'(SEQ ID NO: 8) or the bivalent complex m22(scFv)₂-ETA'(SEQ ID NO: 8).

2. Nucleic acid molecules coding for the recombinant complex designed according to claim 1 for the complete complex.

3. Vectors comprising a nucleic acid molecule of claim 2.

4. Cells other than human embryonic stem cells or non-human organisms synthesising the recombinant complex according to claim 1, obtainable by transforming or transfecting them with nucleic acid molecules of claim 2 or vectors according to claim 3.

5. The organisms or cells according to claim 4, wherein the organisms are prokaryotes, such as *E. coli, B. subtilis,* S. *carnosus,* S. *coelicolor* or *Marinococcus sp.,* lower eukaryotes, such as *Saccharomyces sp., Aspergillus sp., Spodoptera sp.* or *P. pastoris.*

6. medicament comprising a complex according to claim 1, nucleic acid molecules according to claim 2, vectors according to claim 3, isolated cells and/or extracts thereof according to claim 4 or 5, single cell organism, such as prokaryotes and lower eukaryotes or extracts of the organisms according to claims 4 or 5.

7. Use of the complex according to claim 1, and/or of the nucleic acid molecules according to claim 2 and/or the vectors according to claim 3 in the manufacture of a medicament for influencing the cell growth and the physiology of CD64⁺-cells.

8. Use of the complex according to claim 1, or of the nucleic acid molecules coding therefore according to claim 2, or of the vectors according to claim 3 and/or of cells or organisms according to claim 4 or 5 for the preparation of a medicament for the treatment of acute myeloid leukemia, chronic inflammation reactions, such as, emphysema, intrinsic and extrinsic asthma, atopic dermatitis, polymorphic light eruption, SLE, graft versus host, multiple sclerosis, macrophage activation syndrome, rheumatoid arthritis, juvenile arthritis, Crohn's disease and chronic bowel disease.

9. A method to produce the recombinant complex according to claim 1 comprising the following steps:
(a) transforming/transfecting cells other than human embryonic stem cells, microorganism such as bacteria, fungi, and/or ciliates, with the nucleic acid molecules or vectors according to claim 2 and/or 3;
(b) culturing the cells, microorganisms and/or organisms of step (a) under conditions suitable for gene expression and protein synthesis in order to accumulate the recombinant complex intracellularly or in the surrounding culture medium; and
(c) isolating and purifying the recombinant complex of step (b).

## Patentansprüche

1. Rekombinanter Komplex, der wenigstens eine Komponente A und wenigstens eine Komponente B aufweist, wobei Komponente A zwei oder mehr Bindungsdomänen für den Zelloberflächenrezeptor CD64 umfasst und Komponente B eine zelltötende Domäne ist, wobei der Komplex der einwertige rekombinante Komplex m22(scFv)₂-ETA' (SEQ ID Nr. 8) oder der zweiwertige Komplex m22(scFv)₂-ETA' (SEQ ID Nr. 8) ist.

2. Nucleinsäuremoleküle, die für den rekombinanten Komplex, der gemäß Anspruch 1 ausgebildet ist, für den gesamten Komplex codieren.

3. Vektoren, die ein Nucleinsäuremolekül gemäß Anspruch 2 umfassen.

4. Zellen, die keine humanen embryonalen Stammzellen sind, oder nichthumane Organismen, die den rekombinanten Komplex gemäß Anspruch 1 synthetisieren und durch Transformieren oder Transfizieren von Ausgangszellen oder -organismen mit Nucleinsäuremolekülen gemäß Anspruch 2 oder Vektoren gemäß Anspruch 3 erhältlich sind.

5. Organismen oder Zellen gemäß Anspruch 4, wobei die Organismen Prokaryonten, wie *E. coli, B. subtilis, S. carnosus, S. coelicolor* oder *Marinococcus* sp., niedere Eukaryonten, wie *Saccharomyces* sp., *Aspergillus* sp., *Spodoptera* sp. oder *P. pastoris* sind.

6. Medikament, umfassend einen Komplex gemäß Anspruch 1, Nucleinsäuremoleküle gemäß Anspruch 2, Vektoren gemäß Anspruch 3, isolierte Zellen gemäß Anspruch 4 oder 5 und/oder Extrakte davon, einzellige Organismen, wie Prokaryonten und niedere Eukaryonten, oder Extrakte der Organismen gemäß Anspruch 4 oder 5.

7. Verwendung des Komplexes gemäß Anspruch 1 und/oder der Nucleinsäuremoleküle gemäß Anspruch 2 und/oder der Vektoren gemäß Anspruch 3 bei der Herstellung eines Medikaments zur Beeinflussung des Zellwachstums und die Physiologie von CD64-positiven Zellen.

8. Verwendung des Komplexes gemäß Anspruch 1 oder der Nucleinsäuremoleküle gemäß Anspruch 2 oder der Vektoren gemäß Anspruch 3 und/oder der Zellen oder Organismen gemäß Anspruch 4 oder 5 zur Herstellung eines Medikaments zur Behandlung von akuter myeloischer Leukämie, chronischen Entzündungsreaktionen, wie Emphysem, intrinsisches und extrinsisches Asthma, atopischer Dermatitis, polymorpher Lichtdermatose, SLE, Graft-versus-Host-Reaktion, multipler Sklerose, Makrophagenaktivierungssyndrom, rheumatoider Arthritis, juveniler Arthritis, Morbus Crohn und chronischer Darmerkrankung.

9. Verfahren zur Herstellung des rekombinanten Komplexes gemäß Anspruch 1, das die folgenden Schritte umfasst:
(a) Transformieren/Transfizieren von Zellen, die keine humanen embryonalen Stammzellen sind, von Mikroorganismen, wie Bakterien, Pilzen und/oder Ciliaten, mit den Nucleinsäuremolekülen oder Vektoren gemäß Anspruch 2 und/oder 3;
(b) Kultivieren der Zellen, Mikroorganismen und/oder Organismen von Schritt (a) unter Bedingungen, die für die Genexpression und Proteinsynthese geeignet sind, um den rekombinanten Komplex intrazellulär oder im umgebenden Kulturmedium anzureichern; und
(c) Isolieren und Reinigen des rekombinanten Komplexes von Schritt (b).

## Revendications

1. Complexe recombiné ayant au moins un composant A, au moins un composant B, dans lequel le composant A comprend deux ou plusieurs domaines de liaison pour les récepteurs de surface cellulaires CD64, et le composant B est un domaine tueur de cellule, dans lequel le complexe est le complexe recombiné monovalent m22(scFv)₂-ETA'(SEQ ID NO : 8) ou le complexe bivalent m22(scFv)₂-ETA'(SEQ ID NO : 8).

2. Molécules d'acide nucléique codant pour le complexe recombiné défini selon la revendication 1 pour le complexe complet.

3. Vecteurs comprenant une molécule d'acide nucléique de la revendication 2.

4. Cellules autres que les cellules souches embryonnaires humaines ou d'organismes non humains synthétisant le complexe recombiné selon la revendication 1, pouvant être obtenues en les transformant ou les transfectant avec les molécules d'acide nucléique de la revendication 2 ou les vecteurs selon la revendication 3.

5. Organismes ou cellules selon la revendication 4, dans lesquels les organismes sont des procaryotes, tels que *E. coli, B. subtilis, S. carnosus, S. coelicolor* ou *Marinococcus sp.,* des eucaryotes inférieurs tels que *Saccharomyces sp., Aspergillus sp. , Spodoptera sp. ou P. pastoris.*

6. Médicament comprenant un complexe selon la revendication 1, les molécules d'acide nucléique selon la revendication 2, les vecteurs selon la revendication 3, les cellules isolées et/ou extraits de celles-ci selon la revendication 4 ou 5, un organisme cellulaire individuel, tel que les procaryotes, et les eucaryotes inférieurs ou des extraits des organismes selon les revendications 4 ou 5.

7. Utilisation du complexe selon la revendication 1, et/ou des molécules d'acide nucléique selon la revendication 2 et/ou des vecteurs selon la revendication 3 dans la fabrication d'un médicament pour influencer la croissance cellulaire et la physiologie des cellules CD64⁺.

8. Utilisation du complexe selon la revendication 1 ou des molécules d'acide nucléique codant pour celui-ci selon la revendication 2, ou des vecteurs selon la revendication 3 et/ou des cellules ou des organismes selon la revendication 4 ou 5 pour la préparation d'un médicament pour le traitement de la leucémie myéloïde aigue, les réactions inflammatoires chroniques, telle que l'emphysème, l'asthme intrinsèque et extrinsèque, la dermite atopique, l'éruption légère polymorphe, le lupus érythémateux systémique, les troubles greffon-hôte, les scléroses multiples, le syndrome d'activation des macrophages, la polyarthrite rhumatoïde, l'arthrite juvénile, la maladie de Crohn et la maladie entérique chronique.

9. Procédé de production du complexe recombiné selon la revendication 1 comprenant les étapes suivantes :
(a) la transformation/transfection de cellules autres que les cellules souche embryonnaires humaines, d'un microorganisme tel que les bactéries, les champignons et/ou les ciliés, au moyen de molécules d'acide nucléique ou de vecteurs selon la revendication 2 et/ou 3 ;
(b) la culture des cellules, des microorganismes et/ou des organismes de l'étape (a) dans des conditions appropriées pour l'expression génétique et la synthèse des protéines afin d'accumuler le complexe recombiné à l'intérieur des cellules ou dans le milieu de culture environnant ; et
(c) l'isolation et la purification du complexe recombiné de l'étape (b).
